(19) Europäisches Patentamt
European Patent Office
Office européen des brevets

(11) EP 2 799 075 A1

(12) EUROPEAN PATENT APPLICATION
published in accordance with Art. 153(4) EPC

(43) Date of publication:
**05.11.2014 Bulletin 2014/45**

(21) Application number: **12863697.4**

(22) Date of filing: **27.12.2012**

(51) Int Cl.:
***A61K 31/7024*** *(2006.01)* ***A23L 1/30*** *(2006.01)*
***A23L 1/305*** *(2006.01)* ***A23L 2/52*** *(2006.01)*
***A61K 8/60*** *(2006.01)* ***A61K 8/97*** *(2006.01)*
***A61K 36/00*** *(2006.01)* ***A61K 36/18*** *(2006.01)*
***A61P 3/10*** *(2006.01)* ***A61P 13/12*** *(2006.01)*
***A61P 17/16*** *(2006.01)* ***A61P 25/02*** *(2006.01)*
***A61P 27/02*** *(2006.01)* ***A61P 43/00*** *(2006.01)*
***A61Q 19/08*** *(2006.01)* ***C07H 13/08*** *(2006.01)*

(86) International application number:
**PCT/JP2012/083992**

(87) International publication number:
**WO 2013/100105 (04.07.2013 Gazette 2013/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.12.2011 JP 2011285694**

(71) Applicant: **Morishita Jintan Co., Ltd.**
**Osaka-shi**
**Osaka 540-8566 (JP)**

(72) Inventors:
- **NISHIDA, Norihisa**
  **Osaka-shi**
  **Osaka 540-8566 (JP)**
- **NAGATOMO, Akifumi**
  **Osaka-shi**
  **Osaka 540-8566 (JP)**
- **ITO, Hideyuki**
  **Okayama-shi**
  **Okayama 700-0815 (JP)**

(74) Representative: **Schlich, George**
**Schlich**
**9 St Catherine's Road**
**Littlehampton, West Sussex BN17 5HS (GB)**

# (54) MAILLARD REACTION INHIBITOR

(57) Provided are a Maillard reaction inhibitor which effectively inhibits the progress of a Maillard reaction in a living body, and can be safely applied without any adverse side effects and be manufactured without requiring any complicated process, and a skin anti-aging agent, an anti-diabetic complication agent and foods and beverages using the same. The Maillard reaction inhibitor of the present invention contains 50 to 90% by mass of polyphenol as an active ingredient. Since the Maillard reaction inhibitor of the present invention has a potent Maillard reaction inhibitory activity to efficiently inhibit the Maillard reaction in a living body and allow for the prevention and improvement of the various dysfunction of protein in a living body. Due to this activity, the Maillard reaction inhibitor of the present invention suppresses aging and is useful for the prevention and treatment of diabetic complications. Furthermore, when the above-described tannin is mixed in foods and beverages containing collagen, it is possible to suppress the Maillard reaction in the foods and beverages to suppress the deterioration of the foods and beverages, and to also suppress the Maillard reaction in a living body.

[Figure 1]

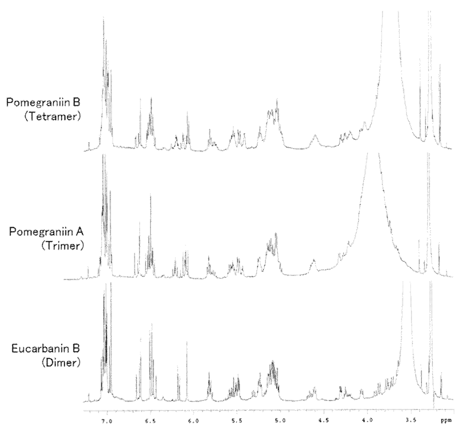


EP 2 799 075 A1

**Description**

Technical Field

The present invention relates to Maillard reaction inhibitors, and more specifically, to Maillard reaction inhibitors that can be used as a material to be mixed in products such as drugs, cosmetics, foods and beverages.

Background Art

A Maillard reaction (glycation reaction) is a reaction in which various intermediates are formed by nonenzymatic and irreversible reactions of reducing sugar and protein through a Schiffs base formation and an Amadori rearrangement, and then, protein glycation end products (advanced glycation end products: AGEs) are produced. The Maillard reaction occurs also in a living body, in which case various biological substances are glycated. The Schiffs base is a reversible compound produced by a reaction of reducing sugar such as glucose and amino groups of protein. Amino groups of lysine, arginine and an N-terminal amino acid of protein are involved in this reaction. An Amadori rearrangement product that is an irreversible compound having a ketoamine structure is produced from a Schiffs base by a rearrangement reaction. The Maillard reaction has three steps, that is, an initial reaction, intermediate formation and final reaction.
3-Deoxyglucosone (3DG), glyoxal (GO) and the like are known as such an intermediate. 3DG is an $\alpha$-dicarbonyl compound produced from an Amadori compound and is involved in production of AGEs. Although AGEs and the intermediates are metabolized by the involvement of some enzymes and excreted, the activity of these enzymes decreases with age and consequently AGEs accumulate in the body.
Carboxymethyllysine (CML), pentosidine, pyrraline, crossline, pyrropyridine, and the like are known as AGEs, and many of these compounds are fluorescent substances (mainly, the excitation wavelength is 370 nm and the fluorescent wavelength is 440 nm). Accumulation of AGEs in tissues and binding of AGEs to their receptors cause a dysfunction of functional protein to develop various symptoms, which become a major factor of a regressive change associated with age. Production and accumulation of AGEs in the skin causes the deterioration of the entire skin such as loss of skin firmness and elasticity, wrinkles, looseness of skin, changes in a skin color, dullness, loss of translucency, and specks on the skin. Moreover, in diabetes patients, AGEs produced by hyperglycemia cause complications such as cataracts, arteriosclerosis, renal functional disorder, and the like.
Accordingly, these various symptoms can be prevented and reduced by inhibiting the Maillard reaction and suppressing the production of AGEs. Recently, various researches and developments to suppress the Maillard reaction (glycation reaction) have been carried out (Patent Documents 1 to 6).

Citation List

Patent Documents

Patent Document 1: Japanese Laid-Open Patent Publication No. 2005-35911
Patent Document 2: Japanese Laid-Open Patent Publication No. 2011-102270
Patent Document 3: Japanese Laid-Open Patent Publication No. 2002-241299
Patent Document 4: Japanese Laid-Open Patent Publication No. 2005-035911
Patent Document 5: Japanese Laid-Open Patent Publication No. 2008-214250
Patent Document 6: Japanese Laid-Open Patent Publication No. 2006-256977


Summary of the Invention

Problem to be Solved by the Invention

The present invention was made in order to solve the foregoing problems, and it is an object thereof to provide a Maillard reaction inhibitor which effectively inhibits the progress of a Maillard reaction in a living body (e.g., human beings, pets, and livestock) and can be safely applied without any adverse side effects and be manufactured without requiring any complicated process, and a skin anti-aging agent, an anti-diabetic complication agent, foods and beverages using the same.

Means for Solving the Problem

The present invention provides a Maillard reaction inhibitor containing 50 to 90% by mass of polyphenol as an active ingredient.

In one embodiment, the polyphenol includes 10 to 50% by mass of ellagitannin with respect to a total mass of the polyphenol.

In a further embodiment, the ellagitannin includes at least one compound selected from the group consisting of punicalin, punicalagin, oenotein B, eucarbanin B, punicacortein C, pomegraniin A and pomegraniin B.

In one embodiment, the polyphenol is contained in the form of a polyphenol containing plant extract.

In one embodiment, the polyphenol containing plant extract is an extract derived from at least one plant selected from the group consisting of plants belonging to the genera *Punica, Quisqualis, Cistus, Terminalia,* and *Combretum.*

The present invention also provides a skin anti-aging agent containing the above Maillard reaction inhibitor.

The present invention also provides an anti-diabetic complication agent containing the above Maillard reaction inhibitor.

The present provides a food or beverage, to which the above Maillard reaction inhibitor is added.

The present invention provides an external preparation, to which the above Maillard reaction inhibitor is added.

Advantageous Effects of the Invention

The Maillard reaction inhibitor of the present invention has a potent Maillard reaction inhibitory activity, effectively suppresses the production of AGEs, and can prevent or improve various symptoms due to AGEs. For example, the Maillard reaction inhibitor can suppress aging due to the Maillard reaction, and prevent and improve wrinkles, looseness of skin and loss of the firmness or elasticity of the skin. Also, the Maillard reaction inhibitor can effectively prevent and treat diabetic complications such as a diabetic neuropathy, diabetic nephropathy and diabetic retinopathy. Moreover, the foods and beverages of the present invention can be easily taken to suppress the occurrence or progress of the Maillard reaction in a living body because the Maillard reaction inhibitor is mixed therein. Furthermore, the foods and beverages of the present invention can also inhibit the occurrence and/or the progress of the Maillard reaction in those foods and beverages by mixing therein the Maillard reaction inhibitor together with collagen, thereby suppressing the deterioration of those foods and beverages to maintain their quality for a long time.

Brief Description of Drawings

FIG. 1 shows $^1$H-NMR spectra for identifying a compound isolated in Example 2: (a) $^1$H-NMR spectrum of pomegraniin B; (b) $^1$H-NMR spectrum of pomegraniin A; and (c) $^1$H-NMR spectrum of eucarbanin B.

Description of Embodiments

The Maillard reaction inhibitor of the present invention contains polyphenol as an active ingredient.

A Maillard reaction inhibitor is also called an antiglycation agent. The Maillard reaction (glycation reaction) is a reaction in which various intermediates are formed by nonenzymatic and irreversible reactions of reducing sugar and protein through a Schiff's base formation and an Amadori rearrangement, and then, protein glycation end products (advanced glycation end products: AGEs) are produced. The Maillard reaction inhibitor of the present invention may inhibit any of the reactions.

In the present invention, the above-described polyphenol is contained at the ratio of 50 to 100% by mass, and preferably 55 to 90% by mass with respect to the total mass of the Maillard reaction inhibitor. If the amount of this polyphenol contained is less than 50% by mass, there is a possibility that the Maillard reaction cannot be sufficiently inhibited in a living body when applied for drugs, cosmetics, foods and beverages described below.

In the present invention, the above-described polyphenol contains ellagitannin. The amount of ellagitannin contained is, for example, 10 to 50% by mass, and preferably 10 to 45% by mass, with reference to the total mass of the polyphenol. Alternatively, the amount of ellagitannin contained is, for example, 5 to 45% by mass, and preferably 10 to 45% by mass, with reference to the total mass of the Maillard reaction inhibitor of the present invention. When ellagitannin is used in such a range of the amount, the Maillard reaction inhibitor of the present invention can effectively inhibit the Maillard reaction in a living body and be efficiently manufactured industrially.

Ellagitannin is a water-soluble compound that, for example, is derived from plants and can react with protein, alkaloids and metal ions to strongly bind thereto and form hardly soluble salts.

Examples of ellagitannin include punicalin represented by the following formula (I):

[Chemical 1]

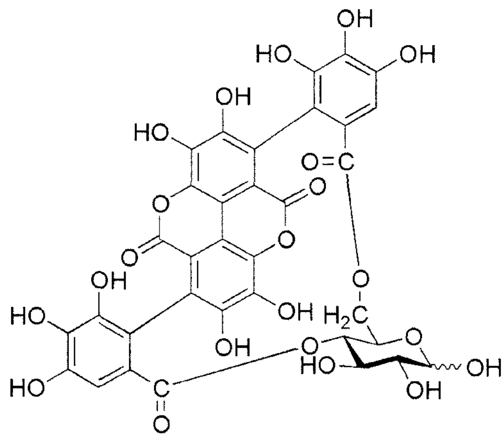

(I)

punicalagin represented by the following formula (II):

[Chemical 2]

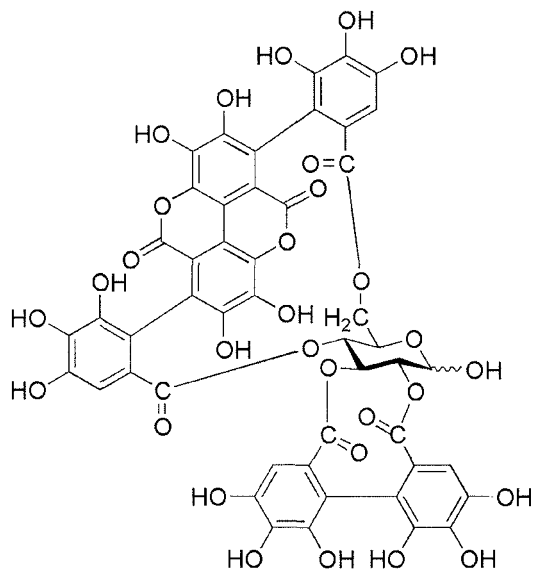

(II)

oenotein B represented by the following formula (III):

[Chemical 3]

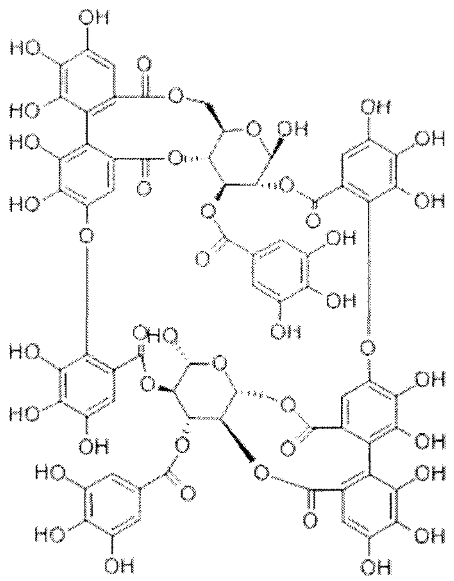

(III)

eucarbanin B represented by the following formula (IV):

[Chemical 4]

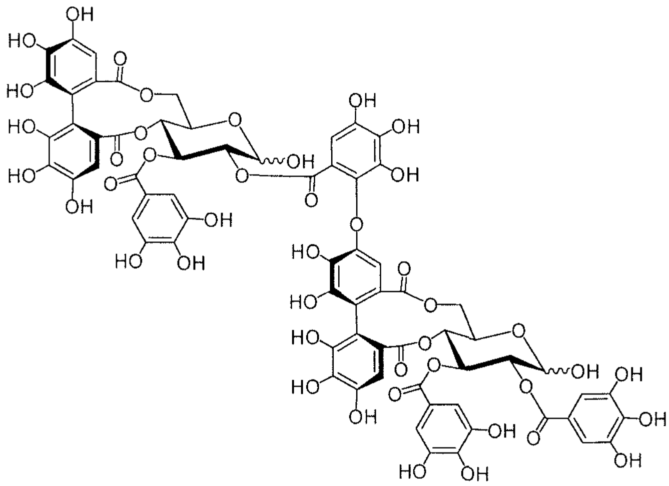

(IV)

punicacortein C represented by the following formula (V):

[Chemical 5]

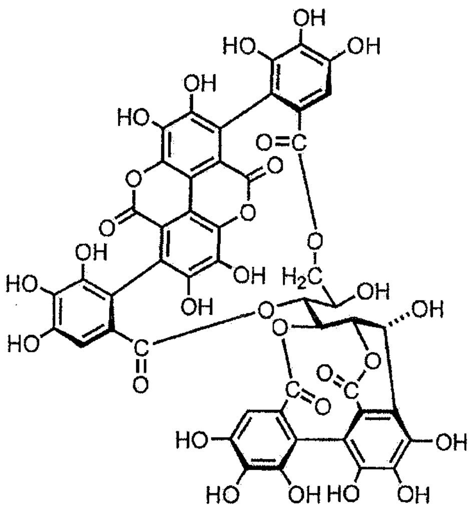

(V)

pomegraniin A represented by the following formula (VI):

[Chemical 6]

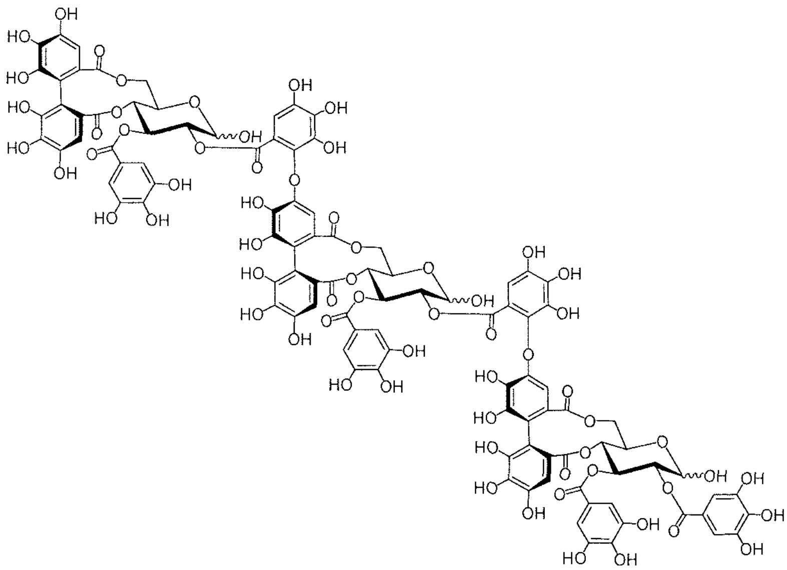

(VI)

pomegraniin B represented by the following formula (VII):

[Chemical 7]

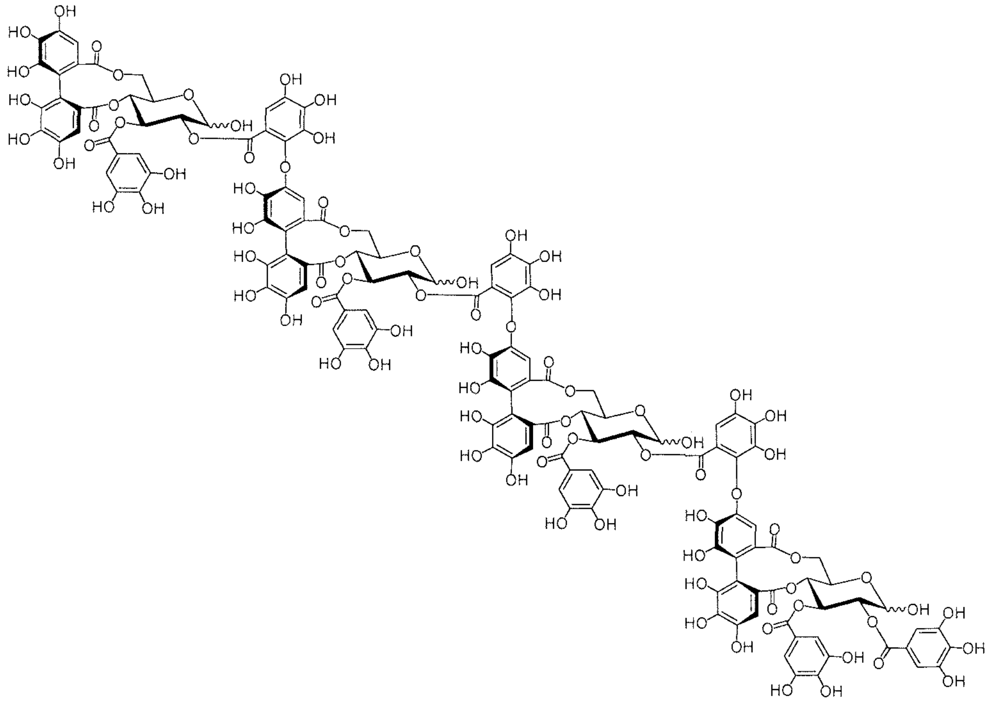

(VII)

and a combination thereof, but are not particularly limited thereto.

In the present invention, ellagitannin may be a single compound itself selected from punicalin, punicalagin, oenotein B, eucarbanin B, punicacortein C, pomegraniin A or pomegraniin B as above or a mixture thereof, or a polymer or a copolymer including at least one of punicalin, punicalagin, oenotein B, eucarbanin B, punicacortein C, pomegraniin A or pomegraniin B as a basic structure. Alternatively, ellagitannin may be a mixture of the single compound and the polymer and/or the copolymer. There is no particular limitation on degree of polymerization of ellagitannin in the form of a polymer or a copolymer, and the degree thereof is 2 to 10.

In the present invention, the above-described polyphenol is preferably contained in form of a polyphenol containing plant extract.

Examples of plants providing the polyphenol containing plant extract include, but are not particularly limited to, plants belonging to the genera *Punica, Quisqualis, Cistus, Terminalia,* and *Combretum.* The polyphenol containing plant extract may be a single extract obtained from the plants or a combination thereof.

The plants belonging to the genus *Punica* belong to Punicaceae. Pomegranate (*Punica granatum*) is preferably used. Examples of parts thereof used for extraction include fruit, trunk bark, branch bark and root bark, and fruit is preferable.

The plants belonging to the genus *Quisqualis* belong to Combretaceae. Rangoon creeper *(Quisqualis indica)* is preferably used. Mature fruit of this plant are called "Shikun-shi" and have been used for medicine over a long period. They are particularly well-known as anthelmintics. Examples of parts thereof used for extraction include fruit and seed.

The plants belonging to the genus *Cistus* belong to Cistaceae. Twenty species belong to the genus *Cistus. Cistus ladaniferus* and *Cistus populifolius* are preferably used.

The plants belonging to the genus *Terminalia* belong to Combretaceae as Rangoon creeper. *Terminalia arborea, Terminalia calamansanai, Terminalia catappa, Terminalia chebula, Terminaria horrida* and *Terminalia triflora* are preferably used.

The plants belonging to the genus *Combretum* also belong to Combretaceae. *Combretum glutinosum* is preferably used.

The plants (whole plant or entire plant) or a portion thereof, processed products thereof (e.g., dried plants, cut plants, chopped plants, or dry powder obtained by pulverizing them, or products obtained by shaping the pulverized dried plants) or crude extracts thereof can be used for the polyphenol containing plant extract. Herein, when merely referring to "plant", processed products of plants (e.g., dried plants, cut plants, chopped plants, or dry powder obtained by pulverizing them, or products obtained by shaping the pulverized dried plants) are also included. Examples of a portion of a plant include, but are not necessarily limited to, parts of a plant such as fruit, fruit skin, flower, stem, leaf, trunk, seed and root. Herein, the "extract" includes liquid extract obtained by being extracted from the above-described plants or processed products thereof with a solvent, a diluted solution or a concentrated solution thereof, or dried products thereof or purified products thereof.

Examples of a solvent used for the extraction of the above-described plants include water, lower alcohol such as ethanol and methanol, lower ester such as ethyl acetate and methyl acetate, acetone and a mixture of these organic solvent and water (hydrous organic solvent). From the viewpoint of further enhancing safety of ingestion by a living body, it is preferable to use water alone, ethanol alone or a mixture of ethanol and water (so-called hydrous ethanol or aqueous solution of ethanol). Furthermore, for example, a 20% (v/v) or more, preferably 30 to 90% (v/v), aqueous solution of ethanol can be used to provide an extract that abundantly contains ellagitannin described above, or effectively provides the inhibition of the Maillard reaction.

There is no particular limitation on extraction conditions (e.g., amount of a solvent, temperature and time). For example, the amount of an extraction solvent is preferably 5 to 50-times the volume per dry mass with respect to pulverized plants, and more preferably 10 to 30-times the volume per dry mass. Although varying with the type of a solvent used, for example, a temperature from a room temperature to a temperature below the boiling point of the solvent used can be adopted as an extraction temperature. An extraction time can vary in accordance with the type, amount, and extraction temperature of the solvent used, and a person skilled in the art can determine a suitable extraction time. When the extraction is performed at room temperature, the extraction time may be, for example, 1 to 48 hours, and preferably 6 to 24 hours, and when the extraction is performed near the boiling point of the solvent, the extraction time may be, for example, approximately 1 to 60 minutes. Furthermore, (a) the extraction may be a single extraction using one extraction solvent; (b) the extraction may be a stepwise extraction using different solvents in which an extraction is performed with one solvent, and another extraction is subsequently performed on the obtained extract with a different solvent; or (c) an extract obtained with one solvent may be combined with an extract obtained with a different solvent. By way of a specific example, the extraction operation (b) can be performed by extracting the above mentioned plant with approximately 20-times the volume of hot water with respect to the dry mass of the plant for about 10 minutes, adding the same amount of ethanol thereto and mixing them, and filtrating the mixture to obtain the filtrate as the extract. It should be noted that such an extraction can be performed under any condition such as still standing, shaking, stirring or refluxing. Alternatively, the extraction is performed by homogenization after the addition of the solvent.

In the present invention, a preferable purification procedure and condition is made by combining, for example, silica gel column chromatography, reversed phase ODS column chromatography, preparative HPLC and the like, using an eluent (e.g., water, organic solvents such as hexane, ethyl acetate, chloroform, methanol and n-butanol, or a mixture thereof) under a preferable condition, but are not particularly limited thereto.

Alternatively, the extract of the above-described plants may be subjected to purification as set forth below in order to enhance the Maillard reaction inhibitory activity. For example, the above-described filtrate obtained by the filtration following the extraction is concentrated by a means commonly used by a person skilled in the art, and then subjected to a column chromatography using preferably a 40 to 90% (v/v), more preferably 50 to 80% (v/v), aqueous solution of $C_1$-$C_3$ alcohol (preferably an aqueous solution of ethanol). An absorbent useful for this column chromatography is preferably an aromatic absorbent, and a specific example thereof is a styrene-divinylbenzene based absorbent. Examples of the styrene-divinylbenzene based absorbent include Amberlite XAD series (manufactured by Organo Corporation). It should be noted that, in the present invention, before the purification using the aqueous solution of $C_1$-$C_3$ alcohol as mentioned above, a preliminary purification may be performed on the above-described column using water (e.g., distilled water) in order to further enhance the Maillard reaction inhibitory activity.

The extract obtained in this manner is used as it is or is concentrated to be used in liquid form, a concentrate, a paste or a dried product obtained by further drying them. Drying is performed by a method commonly used by a person skilled in the art, such as spray drying, lyophilization, vacuum drying and fluidized drying.

It should be noted that, in the present invention, the "extract" also includes a water extract or a solvent extract obtained by the above-described procedure, and a supercritical carbon dioxide extract, solid contents obtained by removing the solvent from these obtained extracts by a method known in the art such as evaporation to dryness under reduced pressure or spray drying, and fractions obtained by further purifying them.

The Maillard reaction inhibitor of the present invention may contain other additives than the above-described polyphenol or polyphenol containing plant extract. Examples of the other additives include cellulose, (cyclo)dextrin, and a combination thereof. There is no particular limitation on the amount of the other additives contained in the present invention, but any amount can be set by a person skilled in the art within the range where the other additives do not inhibit or reduce the above-described Maillard reaction inhibitory effect exhibited by the above-described polyphenol or polyphenol containing plant extract.

The Maillard reaction inhibitor of the present invention is useful as a material constituting, for example, drugs, cosmetics or foods and beverages.

One embodiment of the Maillard reaction inhibitor of the present invention includes anti-aging for the skin of a living body due to the Maillard reaction. For example, the Maillard reaction inhibitor of the present invention can be used to prevent or improve aging of the skin of a living body. Here, aging due to the Maillard reaction means regressive changes resulting from the dysfunction of functional protein due to an uncontrollable reaction of AGEs produced by the Maillard reaction with biological substances. For example, the accumulation of AGEs in the skin reduces skin translucency

and yellows the skin. Although collagen protein plays a roll of maintaining the elasticity of the skin together with elastic fibers, when collagen protein bind to AGEs to form crosslinks therebetween, the movability of collagen is lost and this results in hardening of the skin and wrinkles. The Maillard reaction inhibitor of the present invention can suppress the production of AGEs to suppress such a dysfunction of protein in a living body.

That is, the Maillard reaction inhibitor of the present invention can be used as an active ingredient providing a skin anti-aging agent. The term "skin anti-aging agent" includes a composition capable of exhibiting an anti-aging action on the skin of a living body, such as drugs (including quasi-drugs) and cosmetics.

When the Maillard reaction inhibitor of the present invention is used as a skin anti-aging agent, there is no limitation on routes or methods for administration and ingestion thereof.

For example, when the Maillard reaction inhibitor of the present invention is used as a drug, transcutaneous administration, subcutaneous administration, transvenous administration and oral administration are exemplified. There is no limitation on a dosage form or physical form. Solid, semi-solid and liquid are exemplified, and an injection, an external preparation and an oral preparation are exemplified. A liquid medicine and a dry preparation for being dissolved in use are exemplified as an injection. A patch, an ointment, a plaster, a lotion, a milky lotion, a liquid medicine and suspending agent are exemplified as an external preparation. A powder medicine, a granular medicine, a tablet, a capsule and a liquid medicine (including a suspending agent, an emulsion, and the like) are exemplified as an oral preparation. An external preparation is preferably used for Maillard reaction inhibition in skin tissue.

Drugs may contain an optional ingredient (e.g., a binding agent, a disintegrating agent, a lubricant, a vehicle, a coloring agent, a flavoring agent, a surfactant, a preservative, an antioxidant, an ultraviolet absorbing agent, a moisturizing agent and a pH adjusting agent) other than the Maillard reaction inhibitor of the present invention as mentioned above. Examples of such an optional ingredient include cellulose, (cyclo)dextrin, powdered acacia, methylcellulose, crystalline cellulose, ethylcellulose, polyvinylpyrrolidone, polyethylene glycol, talc, silicon dioxide (e.g., light anhydrous silicic acid or hydrous silicon dioxide), magnesium stearate, stearic acid, stearyl alcohol, starches, hydroxypropyl starch, sodium carboxymethyl starch, powdered agar, carboxymethylcellulose (calcium), low substituted hydroxypropylcellulose, ester oil, wax, higher fatty acid, higher alcohol, anionic surfactant, cationic surfactant, nonionic surfactant, amphoteric surfactant and polyalcohol. In this case, any amount can be set for the contained ingredient by a person skilled in the art within the range where the ingredient does not inhibit or reduce the above-described the Maillard reaction inhibitory effect of the present invention.

When used for cosmetic, cosmetics may contain an optional ingredient (e.g., an oily ingredient such as ester oil, wax, higher fatty acid and higher alcohol; a surfactant such as an anionic surfactant, cationic surfactant, nonionic surfactant and amphoteric surfactant; a moisturizing agent such as polyalcohol; a base ingredient such as water, lower alcohol and silicone; a preservative; an antioxidant; an ultraviolet absorbing agent; a flavor; and a pH adjusting agent) other than the Maillard reaction inhibitor of the present invention as mentioned above. In this case, any amount can be set for the contained ingredient by a person skilled in the art within the range where the ingredient does not inhibit or reduce the above-described the Maillard reaction inhibitory effect of the present invention. The "cosmetics" include base cosmetics such as a face lotion and a milky lotion; an application for the entire face such as a foundation and face powder; and an application for a portion of the face such as an eye-shadow, an eyeliner and a cheek rouge.

The Maillard reaction inhibitor of the present invention can be also used as anti-diabetic complication agent. In diabetes patients, since the concentration of glucose in blood is high, protein in a living body is easily glycated to cause its dysfunction. The Maillard reaction inhibitor of the present invention can suppress the glycation of protein in a living body to prevent or treat diabetic complications. Examples of the diabetic complications include diabetic retinopathy, diabetic nephropathy, diabetic neuropathy, cerebrovascular disorder, ischemic heart disease, diabetic gangrene, hyperlipidemia, chronic infection, cholelithiasis and cataracts.

The present invention also provides foods and beverages to which the above-described Maillard reaction inhibitor is added.

The foods and beverages include feed and pet food, and examples thereof include foods and beverages formulated into a powder, a granule, a tablet, a capsule, a liquid agent, a suspending agent and an emulsion. The foods and beverages may contain other food materials, other active ingredients, and/or additives that can be generally used in the art (e.g., a vehicle such as dextrin, starch, sugars and calcium phosphate, a solvent, a flavor and perfumed oil) other than the above-described Maillard reaction inhibitor.

In the present invention, processed foods, noodles, confectionery, drink, seasonings, powdered milk, health foods, supplements and the like are exemplified as the foods and beverages to which the above-described Maillard reaction inhibitor is added. There is no limitation on a dosage form or physical form of the composition for addition to a food or a beverage as long as it is in the form that is easily added, such as powdery form or liquid form. Although the compositions including a composition, such as a rice seasoning or seasoning, having such a form and amount that can be added to a food or a beverage and mixed therewith by a person who eats or drinks and a composition having a form and amount to be added on processing a food or a beverage varies the form, amount, and degree of processing of the composition in accordance with its addition method or purpose, the composition of the present invention includes all of

them.

Furthermore, by adding the above-described Maillard reaction inhibitor, that is, an ingredient having an inhibitory activity on the Maillard reaction in a living body, the amount of the ingredient contained in the foods and beverages of the present invention can be increased compared with conventional food materials. Therefore, it is possible to ingest, at one time, the inhibition active ingredient with the unexperienced amount in conventional food materials.

The present invention also provides foods and beverages containing collagen and the above-described ellagitannin. The present invention further provides a method for inhibiting the Maillard reaction in foods and beverages by mixing the above-described ellagitannin in foods and beverages containing collagen. The ellagitannin is preferably hydrolyzed tannin. There is no particular limitation on the type and molecular weight of collagen. The foods and beverages may contain sugars that can be generally used in a food field. When the ellagitannin is mixed in the foods and beverages containing collagen, the Maillard reaction of sugars in the foods and beverages with collagen is inhibited, so that it is possible to suppress the deterioration of the foods and beverages and to maintain their quality. Furthermore, after the foods and beverages are ingested, it is possible to suppress the Maillard reaction in a living body. For example, 0.0001 to 100 mg of punicalin and/or punicalagin, preferably 0.001 to 1 mg, is mixed with respect to 1 g of collagen contained in foods and beverages.

The present invention also provides an external preparation containing collagen and the above-described ellagitannin. When used for an external preparation, the external preparation may contain an optional ingredient (e.g., an oily ingredient such as ester oil, wax, higher fatty acid and higher alcohol; a surfactant such as an anionic surfactant, cationic surfactant, nonionic surfactant and amphoteric surfactant; a moisturizing agent such as polyalcohol; a base ingredient such as water, lower alcohol and silicone; a preservative; an antioxidant; an ultraviolet absorbing agent; a flavor; and a pH adjusting agent) other than the above-described Maillard reaction inhibitor of the present invention. In this case, any amount can be set for the contained ingredient by a person skilled in the art within the range where the ingredient does not inhibit or improve the above-described Maillard reaction inhibitory effect of the present invention.

Examples

Next, the present invention will be further described by way of examples, but is not limited to the following examples.

Example 1: Measurement of the Maillard reaction inhibitory activity

A pomegranate extract (containing 50% by mass of polyphenol; manufactured by Morishita Jintan Co., Ltd.), punicalin, punicalagin, oenotein B, eucarbanin B, punicacortein C, pomegraniin A and pomegraniin B were used as samples to be examined. Tannic acid and ellagic acid dihydrate were used for comparison. Aminoguanidine was used as a positive control. These samples were diluted with water to prepare solutions at concentrations of 0.01 μg/mL, 0.03 μg/mL, 0.1 μg/mL, 0.3 μg/mL, 1 μg/mL, 10 μg/mL, 100 μg/mL, 300 μg/mL and 1,000 μg/mL, and the Maillard reaction inhibitory activity of the prepared solutions was measured. The inhibitory activity was measured as follows.

Four reaction solutions A to D shown in Table 1 below were prepared using the above-described prepared solutions of the samples, and were incubated at 60°C for 40 hours to cause the Maillard reaction. Thereafter, 400 μL of the reaction solutions A to D was diluted with 2,400 μL of distilled water and diluted samples were obtained. The fluorescence intensity (the excitation light: 370 nm; the measurement light: 440 nm) of the diluted samples was measured and was expressed as a relative value with respect to the fluorescence intensity of 0.1 μg/mL solution of quinine sulfate that was taken as 100 to calculate the amount of fluorescent products of the Maillard reaction (quantitative value).

[Table 1]

| Sample (Concentration in Reation Solution) | Reaction Solution A | Reaction Solution B | Reaction Solution C | Reaction Solution D |
|---|---|---|---|---|
| Human Serum Albumin Solution | 0.8 mg/mL | 0.8 mg/mL | 0.8 mg/mL | 0.8 mg/mL |
| Glucose Solution | 200 mmol/L | - | 200 mmol/L | - |
| Phosphate Buffer | 50 mmol/L | 50 mmol/L | 50 mmol/L | 50 mmol/L |
| Distilled Water | 100 μL | 200 μL | 200 μL | 300 μL |
| Sample to be Examined | 100 μL | 100 μL | - | - |
| Total Amount | 1000 μL | 1000 μL | 1000 μL | 1000 μL |

Next, the Maillard reaction inhibition ratio (%) with respect to the above-described prepared solutions (concentration (μg/mL)) of the samples was calculated from the obtained quantitative value using the following equation.

[Formula 1]

$$\text{Equation: Inhibition ratio (\%)} = 100 - [(a - b)/(c - d)] \times 100$$

where *a* represents a relative value (-) of the fluorescent products of the Maillard reaction with the reaction solution A calculated in terms of quinine sulfate,

b represents a relative value (-) of the fluorescent products of the Maillard reaction in terms of quinine sulfate when the reaction solution B is used,

c represents a relative value (-) of the fluorescent products of the Maillard reaction in terms of quinine sulfate when the reaction solution C is used, and

d represents a relative value (-) of the fluorescent products of the Maillard reaction in terms of quinine sulfate when the reaction solution D is used.

Thereafter, 50% inhibition ratio ($IC_{50}$) of each sample was calculated as follows. That is, the inhibition ratio was plotted with respect to the sample concentration, 4-parameter plot was performed, and the concentration showing an inhibition ratio of 50% was taken as $IC_{50}$.

Table 2 shows the Maillard reaction inhibition ratios (%) with respect to the prepared solutions. Furthermore, Table 3 shows $IC_{50}$ values calculated from the Maillard reaction inhibition ratios (%) with respect to the prepared solutions shown in Table 2.

[Table 2]

| Maillard reaction inhibition ratio (%) | | | | | | | | | |
|---|---|---|---|---|---|---|---|---|---|
| Conc. (mg/mL) | 0.01 | 0.03 | 0.1 | 0.3 | 1 | 10 | 100 | 300 | 1000 |
| Pomegranate extract | - | - | 8.4 | 40.7 | 70.4 | 85.5 | 76.6 | - | - |
| Punicalin | 7.6 | 6.5 | 32.1 | 69.6 | 73.1 | 84.9 | - | - | - |
| Punicalagin | 4.3 | 10.9 | 36.2 | 71.4 | 73.5 | 86.1 | - | - | - |
| Oenotein B | 15.5 | - | | - | 69.2 | 64.6 | - | - | - |
| Eucarbanin B | - | - | 21.2 | - | 72.9 | 79.4 | - | - | - |
| Punicacortein C | - | - | 39.6 | - | 76.8 | 78.2 | - | - | - |
| Pomegraniin A | - | - | 46.6 | - | 76.7 | 95.4 | - | - | - |
| Pomegraniin B | 30.4 | - | 40.3 | - | 74.3 | 82.5 | - | - | - |
| Aminoguanidine | - | - | - | - | - | - | 40.2 | 51.4 | 62 |
| Tannic acid | 12.7 | - | 48.7 | - | 65.1 | 68.8 | - | - | - |
| Ellagic acid dihydrate | 1.9 | 2.2 | 12.0 | 44.8 | 68.3 | 78.4 | - | - | - |
| -: not measured | | | | | | | | | |

[Table 3]

| | $IC_{50}$ (mg/mL) |
|---|---|
| Pomegranate extract | 0.56 |
| Punicalin | 0.16 |
| Punicalagin | 0.15 |
| Oenotein B | 0.43 |
| Eucarbanin B | 0.43 |

(continued)

| | $IC_{50}$ (mg/mL) |
|---|---|
| Punicacortein C | 0.16 |
| Pomegraniin A | 0.13 |
| Pomegraniin B | 0.21 |
| Aminoguanidine | 273.31 |
| Tannic acid | 0.31 |
| Ellagic acid dihydrate | 0.34 |

As shown in Tables 2 and 3, it is found that each of the pomegranate extract, punicalin, punicalagin, oenotein B, eucarbanin B, punicacortein C, pomegraniin A and pomegraniin B had a superior Maillard reaction inhibitory activity to aminoguanidine serving as a positive control. Furthermore, as is clear from Table 2, it is found that punicalin, punicalagin, oenotein B and pomegraniin B were particularly superior in Maillard reaction inhibition ratio (%) to ellagic acid dihydrate and aminoguanidine serving as a positive control even when prepared at lower concentrations. In addition, as is clear from Tables 2 and 3, it is found that all $IC_{50}$ values of punicalin, punicalagin, punicacortein C, pomegraniin A and pomegraniin B were particularly low compared with those of tannic acid, ellagic acid dihydrate and aminoguanidine serving as a positive control, and each of them has a superior Maillard reaction inhibitory activity.

Example 2: Extraction of ellagitannin from pomegranate powder

Seven hundred milliliters of water was added to 300 g of commercially available dry pomegranate powder (made in China). The mixture was allowed to stand with stirring at 50°C for 24 hours, was allowed to cool, and was centrifuged to provide 900 ml of liquid extract. The liquid extract was injected to a column packed with 100 g of Amberlite XAD4 (manufactured by Organo Corporation), 3,000 mL of water was passed through the column, and then, 1,500 mL of a mixed solution of ethanol to water of 8:1 (v:v) was passed through the column. The obtained fractions were concentrated under a reduced pressure, and then, 5 g of cellulose (Avicel, manufactured by Asahi Kasei Corporation) serving as a lyophilization aid was added to the obtained concentrate of the ethanol-water fractions, and the mixture was lyophilized. In this manner, a sample S constituted of powder derived from pomegranate powder was prepared.

Next, the following measurements were performed on the sample S obtained as described above.

Total amount of polyphenol

The amount of the contained polyphenol was measured as ethyl gallate equivalence according to Folin-Denis method ("Explanation on Analytic Manual of Food Recorded in 5th Revised Standard Table of Food Composition in Japan", p.254, 2001, Chuohoki Publishing Co., Ltd.). That is, a sample solution was prepared by dissolving the sample in distilled water so as to have a concentration of 15 mg/mL. One milliliter of this sample solution and 1 mL of a Folin reagent (obtained by adding 180 mL of distilled water to 25 g of sodium tungstate, 5 g of phosphomolybdic acid and 12.5 mL of phosphoric acid, boiling the mixture under reflux for 2 hours, and then making the total volume 1 L with distilled water) were mixed and were allowed to stand for 5 minutes. One milliliter of 10% aqueous solution of sodium carbonate was further added thereto, the solution was allowed to stand for 1 hour, and then, the absorbance was measured for the solution at 700 nm. The absorbance measured in the same manner as described above, except that distilled water alone was used instead of the sample solution, was used as a control. Aqueous solutions of ethyl gallate at various concentrations were prepared and were measured in the same manner as described above to make a calibration curve. Then, the amount of polyphenol contained in the sample S obtained as described above was measured.

The total amount of polyphenol contained in the sample S was 72.1±0.6% with respect to the mass of the sample S.

Amount of ellagitannin

The amount of ellagitannin contained in the above-described sample S was determined with HPLC (model number: Inertsil ODS-3; manufactured by GL Sciences Inc.) according to the following condition described in literature (J. Agric. Food Chem., 2009, 57(16), p.7395).

<Conditions of HPLC analysis>

Detector: Ultraviolet absorption spectrometer (380 nm)
Column: Inertsil ODS-3 (5 μm, 4.6 x 250 mm) (manufactured by GL Sciences Inc.)
Column temperature: 40°C
Flow rate: 1.0 mL/minute
Injection amount: 25 μL
Mobile phase condition: a linear gradient with 0.5% phosphoric acid (A) and acetonitrile (B) under the following conditions:

| | A | B |
|---|---|---|
| 0 min. | 95% | 5% |
| 10 min. | 85% | 15% |
| 30 min. | 75% | 25% |
| 35 min. | 95% | 5%. |

Table 4 shows the obtained results.

[Table 4]

| Compound | Elution time/min. |
|---|---|
| Punicalin | 7.3 |
| Punicalagin | 11.2, 12.9 |
| Oenotein B | 12.1 |
| Eucarbanin B | 13.1 |
| Punicacortein C | 12.5 |
| Pomegraniin A | 12.3 |
| Pomegraniin B | 13.3 |
| Ellagic acid dihydrate | 24.3 |

As shown in Table 4, it is found that the sample S obtained in this example contained polyphenol, specifically certain amounts of various ellagitannins.

Moreover, the above-described sample S was subjected to the separation according to the following conditions to isolate oenotein B, eucarbanin B, punicacortein C, pomegraniin A and pomegraniin B. Of these compounds, pomegraniin A, pomegraniin B and eucarbanin B were identified as these compounds by $^1$H-NMR spectrum measurement (600 MHz, acetone-$d_6$+$D_2O$) as shown in FIG. 1. $^1$H-NMR data (600 MHz, acetone-$d_6$+$D_2O$) of pomegraniins A and B are shown below: 6.97-7.05 (galloyl-H), 6.11-6.67 (HHDP-H, valoneoyl-H), 5.55-5.85 (glucose H-3), 5.49-5.61 (glucose H-1 (α-anomer), H-3), 5.24-5.26 (glucose H-6), 5.03-5.22 (glucose H-1 (ß-anomer), H-2, 4, 6), 4.61-4.63 (glucose H-5 (ß-anomer)), 4.30-4.32 (glucose H-1 (ß-anomer)), 4.08-4.26 (glucose H-5 (α-anomer)), 3.66-3.93 (glucose H-6). In addition, results of electrospray mass spectrometry (ESI-MS) of pomegraniins A and B are also shown below:

Pomegraniin A (trimer) ESI-MS: m/z 2353 (M-H)-, 1176 $(M-2H)^{2-}$
Pomegraniin B (tetramer) ESI-MS: m/z 3137 $(M-H)^-$, 1568 $(M-2H)^{2-}$.

<Conditions of HPLC analysis>

Detector: Ultraviolet absorption spectrometer (280nm)
Column: YMC-Pack SIL A-003 (4.6 x 250 mm) (manufactured by YMC Co., Ltd.)
Column temperature: 25°C
Flow rate: 1.5 mL/minute
Mobile phase condition: n-hexane:MeOH:THF:HCOOH=47:39:13:1 + $(COOH)_2$ 450 mg/L

Example 3: Anti-aging action on skin

Effect of the pomegranate extract on the collagen crosslink formation by a glycation reaction was evaluated using a collagen gel contraction activity of human dermal fibroblasts as an index. That is, collagen gel was prepared in a 12-well cell culture plate using a collagen gel culture kit (Cellmatrix, manufactured by Nitta Gelatin Inc.).

The samples S obtained in Example 2 at various concentrations were prepared using a 10 mM solution of glucose-6-phosphate, were added on the collagen gel, and were then incubated at 37°C for 10 days so as to cause the glycation reaction. Unreacted glucose-6-phosphate was washed away, and $1x10^5$ cells/mL of fibroblasts were seeded on the collagen gel and cultured in DMEM medium containing 0.25% FBS. After 3 hours, the collagen gel was peeled off the wall of the well and collagen was contracted. After 48 hours, the medium was sucked and removed, the diameter of the collagen gel was measured, and the area of the collagen gel was calculated.

As Control 1, the area of the collagen gel was calculated in the same manner as described above, except that the above-described solution of glucose-6-phosphate was not added and the glycation reaction was not caused. In addition, as Control 2, the area of the collagen gel was calculated in the same manner as described above, except that the sample S obtained in the above-described Example 2 was not added. The area calculated from Control 1 was denoted as A, the area calculated from Control 2 was denoted as B, the area calculated from the sample was denoted as C, and the inhibition ratio was calculated from the following equation.

[Formula 2]

$$\text{Inhibition ratio (\%)} = [(C - A)/(B - A)]x100$$

Table 5 shows the obtained results.

[Table 5]

| Test Material | Concentration (mg/mL) | Glycation reaction | Area of Collagen gel ($cm^2$) | Inhibition rate(%) |
|---|---|---|---|---|
| Control 1 | 0.0 | absence | 2.47±0.12 | - |
| Control 2 | 0.0 | presence | 3.25±0.15 | - |
| Sample S of Example 2 | 0.01 | presence | 3.22±0.22 | 3.8 |
| | 0.03 | presence | 3.14±0.19 | 14.1 |
| | 0.1 | presence | 2.90±0.25 | 44.9 |
| | 0.3 | presence | 2.74±0.24 | 65.4 |
| | 1.0 | presence | 2.70±0.21 | 70.5 |
| | 3.0 | presence | 2.65±0.18 | 76.9 |
| -: not calculated | | | | |

As shown in Table 5, the higher the added concentration of the sample S obtained in Example 2 was, the smaller the area of the collagen gel was and the higher contraction activity was exhibited. Therefore, the sample S exhibited a superior anti-aging action on the skin.

Example 4: Effect on diabetic complications

Male TSOD (Tsumura, Suzuki, Obese Diabetes) mice with spontaneous development of obesity and diabetes were housed until 10 weeks old, and individuals in which the appearance of urinary sugar was confirmed were used for the experiment. Male TSNO (Tsumura, Suzuki, Non Obesity) mice at the same age were used as normal control mice with no development of obesity and diabetes. The mice were divided into two groups such that the averages of the blood sugar level and bodyweight were uniform. The first group was fed with a normal diet, the second group was fed with a normal diet in which the powdered sample S obtained in Example 2 was mixed at a ratio of 1%, and both groups were housed for 12 weeks. The normal control mice were fed with the normal diet and were housed for 12 weeks in the same manner. The mice freely drank tap water as drinking water.

After the administration for 12 weeks was finished, blood of all the mice was collected from the abdominal aorta under anesthesia, the mice were put to death by exsanguination, and the thoracic aorta was extracted. In addition, the eyeball was extracted and the crystalline lens was collected. The blood sample was centrifuged to collect blood plasma, and the CML concentration was measured by ELISA. After fat was removed, the extracted aorta was lyophilized and was pulverized finely. A portion thereof was treated with a protease and was centrifuged to collect the supernatant. This supernatant was measured at 370 nm of the excitation wavelength and 430 nm of the fluorescent wavelength, and the amount of AGE was calculated. The crystalline lens was homogenized and was centrifuged, and the amount of AGE contained in the supernatant was measured by ELISA.

Tables 6 to 8 show the obtained results.

[Table 6]

CML in blood

| Absorbance (450nm) | Absorbance(-) | SE |
|---|---|---|
| TSNO | 0.52 | 0.14 |
| TSOD | 1.13 | 0.21 |
| TSOD (After ingested a sample S of Example 2) | 0.84 | 0.11 |

[Table 7]

AGE in Aorta

| Fluoresence | Fluoresence(-) | SE |
|---|---|---|
| NO | 7543 | 650 |
| TSOD | 9620 | 745 |
| TSOD (After ingested a sample S of Example 2) | 8460 | 469 |

[Table 8]

AGE in Crystalline

| Absorbance (450nm) | Absorbance(-) | SE |
|---|---|---|
| TSNO | 0.65 | 0.18 |
| TSOD | 1.27 | 0.32 |
| TSOD (After ingested a sample S of Example 2) | 1.04 | 0.36 |

As shown in Tables 6 to 8, in the TSOD mice ingesting the sample S obtained in Example 2, the formation of AGE in the blood, aorta and crystalline lens was suppressed compared with the case of TSOD mice ingesting only the normal diet. Therefore, it is possible that the sample S obtained in Example 2 is useful for the prevention or delay of development of the diabetic complications.

Example 5: Preparation of a beverage

A beverage was prepared based on the following formula.

| Ingredients | Compounding ratio (Mass ratio) |
|---|---|
| Glycerin | 10.0 |
| Sample 2 obtained in Example 2 | 1.0 |
| Cellulose | 0.1 |
| Citric acid | 0.3 |
| Flavor | 0.1 |

(continued)

| Ingredients | Compounding ratio (Mass ratio) |
|---|---|
| Purified water | Remaining quantity |

The above-described ingredients were mixed together and were stirred to produce a beverage.

Example 6: Preparation of face lotion

The following ingredients were uniformly mixed at the following ratio to provide a face lotion.

| Ingredients | Compounding ratio (Mass ratio) |
|---|---|
| Glycerin | 10.0 |
| 1,3-butylene glycol | 6.0 |
| Sample 2 obtained in Example 2 | 1.0 |
| Citric acid | 0.1 |
| Sodium citrate | 0.3 |
| Polyoxyethylene | 1.0 |
| Ethyl alcohol | 8.0 |
| Paraben | 0.1 |
| Flavor | 0.1 |
| Purified water | Remaining quantity |

Industrial Applicability

The Maillard reaction inhibitor of the present invention has a potent Maillard reaction inhibitory activity to efficiently inhibit the Maillard reaction in a living body and allow for the prevention and improvement of the various dysfunction of protein in a living body. Due to this activity, the Maillard reaction inhibitor of the present invention suppresses aging and is useful for the prevention and treatment of diabetic complications. Furthermore, when the above-described tannin is mixed in foods and beverages containing collagen, it is possible to suppress the Maillard reaction in the foods and beverages to suppress the deterioration of the foods and beverages, and to also suppress the Maillard reaction in a living body. The Maillard reaction inhibitor of the present invention is useful in various fields such as drugs, cosmetics and food.

## Claims

1. A Maillard reaction inhibitor containing 50 to 90% by mass of polyphenol as an active ingredient.

2. The Maillard reaction inhibitor according to claim 1, wherein the polyphenol includes 10 to 50% by mass of ellagitannin with respect to a total mass of the polyphenol.

3. The Maillard reaction inhibitor according to claim 2, wherein the ellagitannin comprises at least one compound selected from the group consisting of punicalin, punicalagin, oenotein B, eucarbanin B, punicacortein C, pomegraniin A and pomegraniin B.

4. The Maillard reaction inhibitor according to any one of claims 1 to 3, wherein the polyphenol is contained in the form of a polyphenol containing plant extract.

5. The Maillard reaction inhibitor according to claim 4, wherein the polyphenol containing plant extract is an extract derived from at least one plant selected from the group consisting of plants belonging to the genera *Punica, Quisqualis, Cistus, Terminalia,* and *Combretum.*

6. A skin anti-aging agent containing the Maillard reaction inhibitor according to any one of claims 1 to 5.

7. An anti-diabetic complication agent containing the Maillard reaction inhibitor according to any one of claims 1 to 5.

**8.** A food or beverage, to which the Maillard reaction inhibitor according to any one of claims 1 to 5 is added.

**9.** An external preparation, to which the Maillard reaction inhibitor according to any one of claims 1 to 5 is added.

[Figure 1]

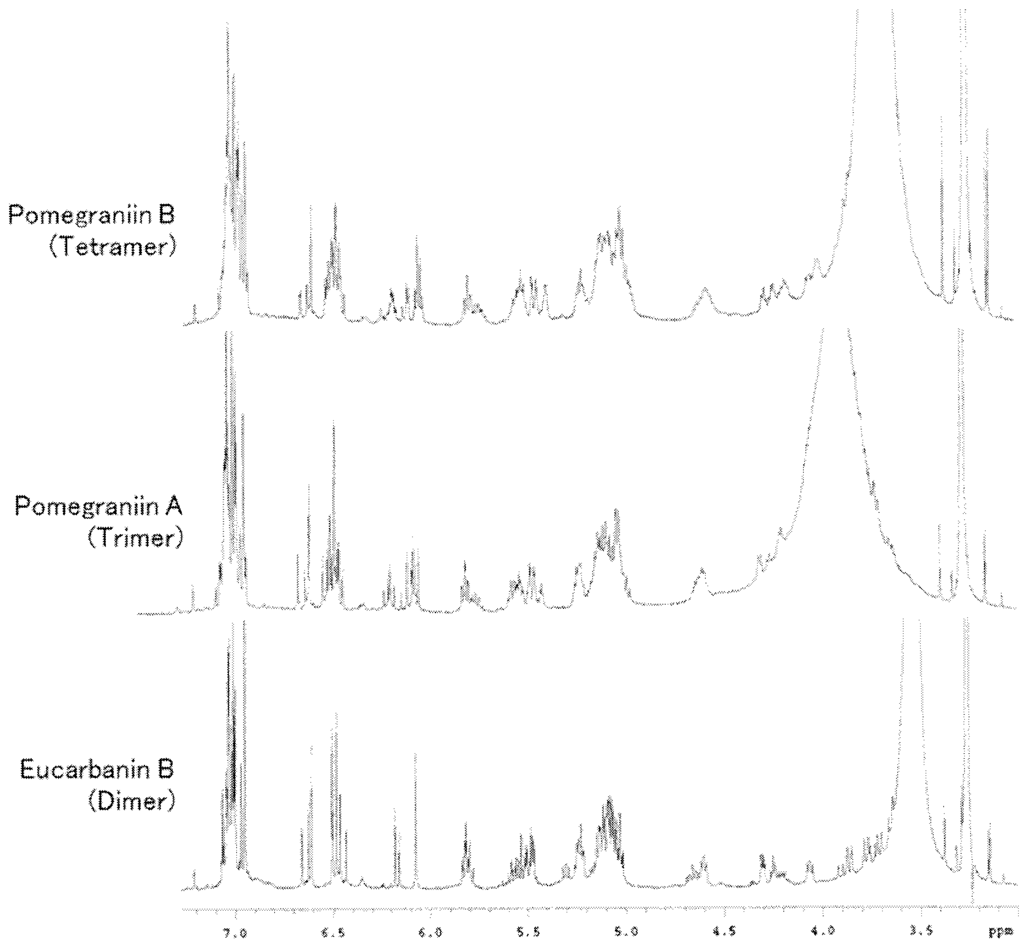
Pomegraniin B
(Tetramer)
Pomegraniin A
(Trimer)
Eucarbanin B
(Dimer)
7.0
6.5
6.0
5.5
5.0
4.5
4.0
3.5
ppm

**INTERNATIONAL SEARCH REPORT**

International application No. PCT/JP2012/083992

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
A23L1/00-2/84, A61K8/00-8/99, 31/33-33/44, 36/00-36/9068, A61P1/00-A61Q90/00, C07H13/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CA/REGISTRY/MEDLINE/EMBASE/BIOSIS(STN), JSTPlus/JMEDPlus/JST7580(JDreamII), G-Search

C. DOCUMENTS CONSIDERED TO BE RELEVANT

| Category* | Citation of document, with indication, where appropriate, of the relevant passages | Relevant to claim No. |
|---|---|---|
| X | JP 2002-241299 A (Ichimaru Pharcos Co., Ltd.), 28 August 2002 (28.08.2002), tests 1, 5; paragraph [0170] (Family: none) | 1-9 |
| X | JP 2008-542283 A (Pom Wonderful, L.L.C.), 27 November 2008 (27.11.2008), paragraphs [0037], [0042] to [0044] & US 2006/0269629 A1 & US 2010/0009019 A1 & US 2010/0173029 A1 & US 2010/0298250 A1 & EP 1901756 A2 & WO 2006/127832 A2 | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 January, 2013 (17.01.13) | 29 January, 2013 (29.01.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No.
PCT/JP2012/083992

C (Continuation). DOCUMENTS CONSIDERED TO BE RELEVANT

| Category* | Citation of document, with indication, where appropriate, of the relevant passages | Relevant to claim No. |
|---|---|---|
| X | JP 2010-534488 A (The State of Israel, Ministry of Agriculture & Rural Development, Agricultural Research Organization, (A.R.O.), Volcani Center), 11 November 2010 (11.11.2010), claims 1, 12, 15, 19; paragraph [0118] & US 2010/0278992 A1 & EP 2190445 A & WO 2009/016620 A2 | 1-9 |
| X | WO 2011/011721 A2 (AMAZENTIS SA), 27 January 2011 (27.01.2011), claims 1, 33 & US 2011/0065662 A1 & EP 2456772 A & JP 2013-500266 A | 1-9 |
| X | JP 2000-143491 A (Pola Chemical Industries Inc.), 23 May 2000 (23.05.2000), claims 1, 3 (Family: none) | 1-9 |
| P,X | JP 2012-121874 A (Maruzen Pharmaceuticals Co., Ltd.), 28 June 2012 (28.06.2012), claim 1; paragraph [0072] (Family: none) | 1-9 |

Form PCT/ISA/210 (continuation of second sheet) (July 2009)

**INTERNATIONAL SEARCH REPORT**

International application No.
PCT/JP2012/083992

Continuation of A. CLASSIFICATION OF SUBJECT MATTER
(International Patent Classification (IPC))

*A61K31/7024*(2006.01)i, *A23L1/30*(2006.01)i, *A23L1/305*(2006.01)i, *A23L2/52*(2006.01)i, *A61K8/60*(2006.01)i, *A61K8/97*(2006.01)i, *A61K36/00*(2006.01)i, *A61K36/18*(2006.01)i, *A61P3/10*(2006.01)i, *A61P13/12*(2006.01)i, *A61P17/16*(2006.01)i, *A61P25/02*(2006.01)i, *A61P27/02*(2006.01)i, *A61P43/00*(2006.01)i, *A61Q19/08*(2006.01)i, *C07H13/08*(2006.01)i

(According to International Patent Classification (IPC) or to both national classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2005035911 A
- JP 2011102270 A
- JP 2002241299 A
- JP 2008214250 A
- JP 2006256977 A

### Non-patent literature cited in the description

- Explanation on Analytic Manual of Food Recorded in 5th Revised Standard Table of Food Composition in Japan. Chuohoki Publishing Co., Ltd, 2001, 254
- *J. Agric. Food Chem.,* 2009, vol. 57 (16), 7395